# EUROPEAN PATENT APPLICATION

(11) **EP 2 361 594 A1**
(43) Date of publication of application: **31.08.2011**
(21) Application number: 09843483.0
(22) Date of filing: 10.07.2009
(51) Int. Cl.: A61F 2/88

(54) **STENT**

(71) Applicant: Taewoong Medical Co., Ltd., Kimpo-si Kyunggi-do 415-873 (KR)
(72) Inventor: SHIN, Kyong Min, Seoul 120-090 (KR); GWON, Dong Il, Kyunggi-do 448-130 (KR)
(74) Representative: Wallin, Nicholas James
(86) International application number: PCT/KR2009/003792
(87) International publication number: WO 2011/004925

(57) **Abstract**

If smooth transportation of bile toward the duodenum is difficult due to cancerous cells growing in the bile duct or due to stricture of the bile duct, a cylindrical stent may be implanted into the stenosed site of the bile duct to enable smooth supply of bile. Conventional stents have no risk of slippage at an implanted position thereof, but suffer from invasively growing cancerous cells. Although a covered stent having a coating has been proposed to solve invasion of the growing cancerous cells, the covered stent may have a risk of slippage at an implanted position thereof and also, may cause pancreatitis and cholelithiasis. To solve the above described problems, disclosed herein is a stent to prevent backflow of food from the duodenum to the bile duct while assuring smooth transportation of bile without a risk of unwanted displacement caused by the transported bile. The stent includes a cylindrical outer stent, and a downwardly tapered inner stent covered with a polytetrafluoroethylene (PTFE) or silicone coating having biocompatibility. The inner and outer stents are coupled to each other to define a double structure.

## Description

### [Technical Field]

The present invention relates to a stent, and more particularly, to a stent having a double structure, which is free from slippage at an implanted position thereof and invasion of a lesion, such as, e.g., a cancerous tumor, and also, when being implanted in the bile duct, may prevent backflow of, e.g., food from the duodenum to the bile duct.

### [Background Art]

In general, in order to prevent stricture of a variety of blood vessels or bodily organs, or to solve deterioration in movement of food, blood or various enzymes caused when, e.g., a cancerous tumor growing in a tubular organ blocks or makes narrow the tubular organ, a stent is usually implanted in the blocked or narrowed tubular organ to forcibly expand the organ for the sake of smooth movement of, e.g., food or blood.

Such a stent is mainly made of a shape memory alloy to have a cylindrical shape. Recently, various kinds of stents having different shapes have been selectively used at different stenting sites of internal organs, blood vessels, and the like.

Conventional stents have no risk of slippage at an implanted position thereof, but suffer from invasively growing cancerous cells. Although a covered stent having a coating has been proposed to solve invasion of the growing cancerous cells, the covered stent may have a risk of slippage at an implanted position thereof and also, may cause pancreatitis and cholelithiasis.

As one example of bodily organs for stenting, the bile duct is a passage to transport digestive enzymes secreted in the liver to the duodenum.

If the bile duct has difficulty in smooth transportation of bile from the liver to the duodenum due to cancerous cells growing in the bile duct or due to stricture of the bile duct, a cylindrical stent may be implanted into the stenosed site of the bile duct to enable smooth supply of the bile.

When the stent is implanted in the bile duct to forcibly expand the interior of the bile duct as described above, however, this may prevent supply of bile because food having passed through the stomach may flow backward to the liver through the stent implanted in the bile duct, rather than passing through the duodenum.

### [Disclosure]

### [Technical Problem]

Therefore, the present invention has been made in view of the above problems, and it is an object of the present invention to provide a stent having a double structure, which is free from slippage at an implanted position thereof and invasion of a lesion, such as, e.g., a cancerous tumor, and also, may prevent backflow of food from the duodenum to the bile duct while assuring smooth transportation of bile without a risk of unwanted displacement caused by the transported bile.

### [Technical Solution]

In accordance with an aspect of the present invention, the above and other objects can be accomplished by the provision of a stent including a cylindrical stent, and a downwardly tapered stent inserted into the cylindrical stent and covered with a polytetrafluoroethylene (PTFE) or silicone material having biocompatibility, the cylinder stent and downwardly tapered stent being coupled to each other to define a double structure.

If necessary, the outwardly located stent may also have a downwardly tapered shape so as not to be pushed toward the duodenum when the stent is implanted into the bile duct.

### [Advantageous Effects]

In accordance with the present invention, as a result of providing a stent with a double structure, an outwardly located stent has the effect of effectively maintaining the stent at an implanted position thereof without a risk of slippage, and an inwardly located covered stent has the effect of preventing invasion of a lesion, such as a cancerous tumor.

When the stent is inserted into the bile duct, the outwardly located stent having a cylindrical or downwardly tapered shape is placed on the inner wall of the bile duct, thus serving to expand the bile duct, and the inwardly located stent having a downwardly tapered shape serves to prevent backflow of, e.g., food from the duodenum to the liver through the bile duct while assuring smooth transportation of bile.

### [Description of Drawings]

The above and other objects, features and other advantages of the present invention will be more clearly understood from the following detailed description taken in conjunction with the accompanying drawings, in which:
FIG. 1 is an exploded perspective view illustrating a stent in accordance with one exemplary embodiment of the present invention;
FIG. 2 is a partial sectional view illustrating one coupling example of an outer stent and an inner stent in accordance with the present invention;
FIG. 3 is a partial sectional view illustrating another coupling example of the outer stent and the inner stent in accordance with the present invention;
FIG. 4 is a reference view illustrating implantation of the stent in accordance with the present invention; and
FIG. 5 is a view illustrating another exemplary embodiment of the present invention.

### <Description of reference numerals related to important parts of the drawings>

Cylindrical outer stent : 1 P TFE or silicone coating : 2
Inner stent having a downwardly tapered funnel shape : 3

### [Best Mode]

In accordance with an embodiment of the present invention, a stent includes an outer stent 1, which is made of a shape memory alloy and has a cylindrical mesh shape, and an inner stent 3 which has a downwardly tapered funnel shape and is covered with a polytetrafluoroethylene (PTFE) or silicone coating 2. The outer stent 1 and the inner stent 3 located inside the outer stent 1 are coupled to each other to define a double structure.

### [Mode for Invention]

Hereinafter, the present invention will be described in detail with reference to the accompanying drawings.

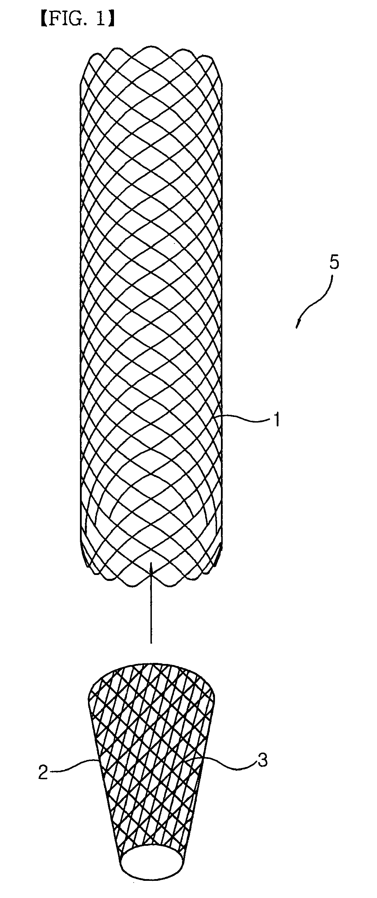

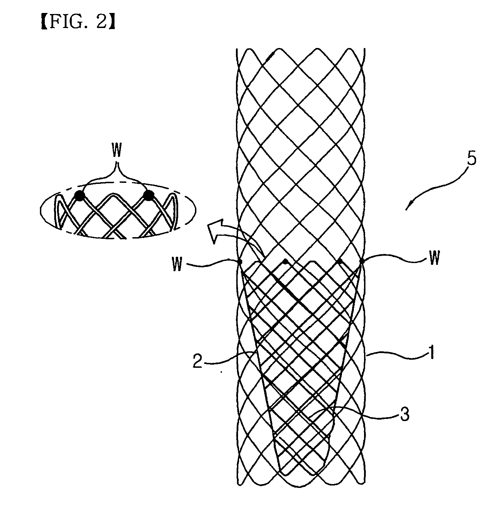

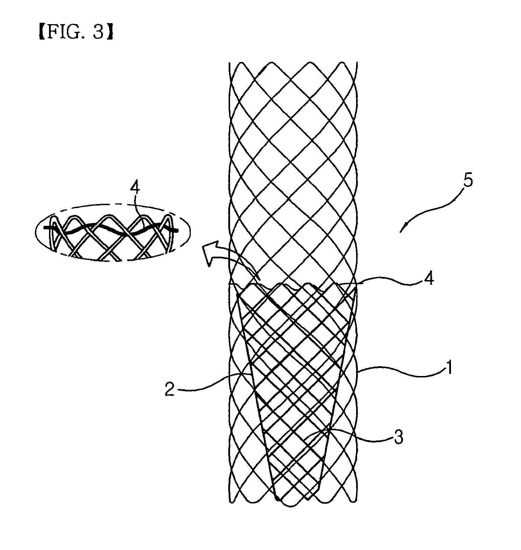

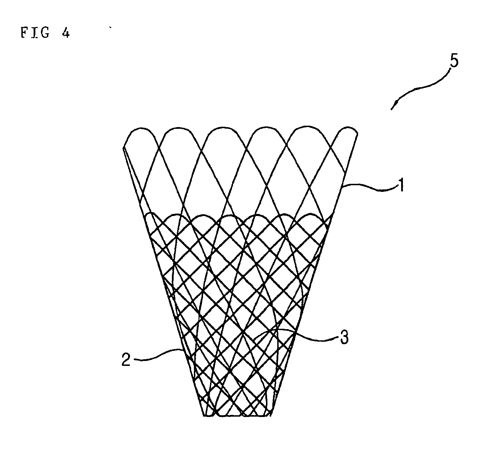

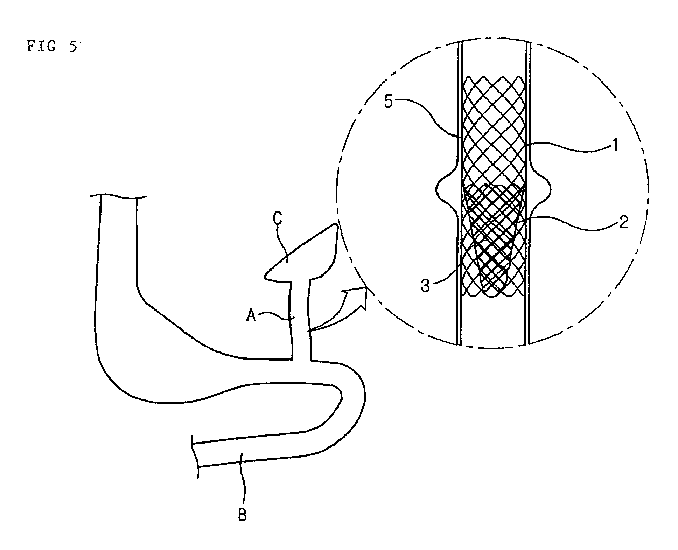

FIG. 1 is an exploded perspective view illustrating a stent in accordance with one exemplary embodiment of the present invention, FIG. 2 is a partial sectional view illustrating one coupling example of an outer stent and an inner stent in accordance with the present invention, FIG. 3 is a partial sectional view illustrating another coupling example of the outer stent and the inner stent in accordance with the present invention, FIG. 4 is a reference view illustrating implantation of the stent in accordance with the present invention, and FIG. 5 is a view illustrating another exemplary embodiment of the present invention.

The stent in accordance with the present invention includes an outer stent 1, which has a cylindrical shape and is made of a shape memory alloy, and an inner stent 3 which has a downwardly tapered funnel shape and is covered with a polytetrafluoroethylene (PTFE) or silicone coating 2.

In the stent 5 having a double structure consisting of the inner stent 3 and outer stent 1 in accordance with the present invention, the inner stent 3 is located inside the outer stent 1 and is coupled to the outer stent 1.

In this case, to accomplish the coupling of the outer stent 1 and the inner stent 3, the outer stent 1 and the inner stent 3 may be welded to form weld spots W, or may be connected to each other using a shape memory alloy wire or thread 4.

The outer stent 1, which serves as an outwardly located component of the stent in accordance with the present invention, has a mesh shape, so as to be inserted into and caught by a partial region of the inner wall of the organ once implanted in the organ by, e.g., angiography. In this way, the stent of the present invention has no risk of slippage at an implanted position thereof.

In addition, since the inner stent 3 is covered with the PTFE or silicone coating 2 as described above, the inner stent 3 may prevent invasion of a lesion, such as, e.g., a cancerous tumor even if expansion of the lesion occurs after implantation of the stent.

In this way, the stent of the present invention has no risk of slippage at the implanted position thereof and also, is free from invasion of the lesion, such as, e.g., a cancerous tumor, despite expansion of the lesion.

The above described stent 5 in accordance with the present invention has the effect of preventing backflow. This will now be described with reference to FIG. 3 in relation to stenting for a bile duct A using angiography.

The outer stent 1 is positioned at an inner wall of the bile duct A and functions to expand the bile duct A. The inner stent 3 located inside the outer stent, which has a downwardly tapered funnel shape and is coated with the PTFE or silicone coating 2, functions to assist digestive enzymes secreted in a liver C to be smoothly transported downward from the bile duct A toward a duodenum B through the inner stent 3.

The inner stent 3, which has a downwardly tapered funnel shape and is covered with the PTFE or silicone coating 2, is located inside the outer stent 1 such that an upper end of the inner stent 3 is connected to an inner position of the outer stent 1 even through, e.g., food inside the duodenum B flows backward toward the bile duct A through the outer stent 1. In addition, the outer stent 1 comes into close contact with the inner wall of the bile duct A and consequently, may effectively prevent the backflow of, e.g., food toward the liver C. The inner stent 3, a lower end of which defines a narrow passage, further effectively prevents the backflow of food thereinto.

In the present invention, the outer stent 1 is inserted into the bile duct A to expand the bile duct A. In this case, in consideration of the fact that the outer stent 1 is implanted in the inner wall of the bile duct A, the outer stent 1 may have a downwardly tapered shape so as not to be pushed toward the duodenum B by digestive enzymes secreted in the liver C.

Further, in the present invention, as the upper end of the inner stent 3 is coupled to the outer stent 1, the lower end of the inner stent 3 may be moved by, e.g., food passing through the duodenum B. Thus, the inner stent 3 may be free from backflow of, e.g., food from the duodenum B.

Although the inner stent 3 and the outer stent 1 are described as being connected to each other by use of the shape memory alloy wire or thread 4, the coupling of the inner stent 3 and the outer stent 1 may be performed by welding as occasion demands.

As described above, since the mesh shaped outer stent may prevent slippage of the stent at an implanted position thereof and the inner stent is free from invasion of any lesion, such as a cancerous tumor, the stent of the present invention is applicable to, e.g., the organs or blood vessels. Furthermore, owing to the anti-backflow function thereof, the stent of the present invention may be implanted in any organs having a risk of backflow, such as the alimentary canal, as well as the bile duct.

It will be appreciated that the bile duct application of the present invention is given only by way of example, and the scope of the present invention is not limited thereto.

## Claims

1. A stent comprising:
an outer stent made of a shape memory alloy and having a cylindrical mesh shape; and
an inner stent having a downwardly tapered funnel shape and covered with a polytetrafluoroethylene (PTFE) or silicone coating,
wherein the outer stent and the inner stent located inside the outer stent are coupled to each other to define a double structure.

2. The stent according to claim 1, wherein the inner stent has an upper end coupled to the outer stent.

3. The stent according to claim 1, wherein the outer stent has a downwardly tapered shape.

4. The stent according to claim 1 or 2, wherein the coupling of the inner stent and the outer stent is performed by welding.

5. The stent according to claim 1 or 2, wherein the coupling of the inner stent and the outer stent is performed by use of a wire or thread.
